# EUROPEAN PATENT APPLICATION

(11) **EP 4 506 451 A1**
(43) Date of publication of application: **12.02.2025**
(21) Application number: 23902729.5
(22) Date of filing: 13.12.2023
(51) Int. Cl.: C12N 9/10, C12N 15/54, C12N 15/70, C12N 1/21, C12P 17/12, C12R 1/19

(54) **OMEGA-TRANSAMINASE MUTANT AND USE THEREOF**

(30) Priority: 16.12.2022 CN 202211623314
(71) Applicant: Zhejiang Yongtai Technology Co., Ltd., Taizhou, Zhejiang 317016 (CN); Zhejiang University of Technology, Hangzhou, Zhejiang 310014 (CN); Zhejiang Yongtai Pharmaceutical Co., Ltd., Taizhou, Zhejiang 317016 (CN); Zhejiang Yongtai Chiral Medicine Technology Co., Ltd., Taizhou, Zhejiang 317016 (CN)
(72) Inventor: CHENG, Feng, Hangzhou, Zhejiang 310014 (CN); HE, Kuang, Hangzhou, Zhejiang 310014 (CN); LIU, Zhiqiang, Hangzhou, Zhejiang 310014 (CN); ZHENG, Yuguo, Hangzhou, Zhejiang 310014 (CN); XIA, Haijian, Hangzhou, Zhejiang 310014 (CN); GU, Jianxiong, Hangzhou, Zhejiang 310014 (CN); LIN, Jiaohua, Hangzhou, Zhejiang 310014 (CN); JIN, Yizhong, Hangzhou, Zhejiang 310014 (CN)
(74) Representative: Garavelli, Paolo
(86) International application number: PCT/CN2023/138290
(87) International publication number: WO 2024/125533

(57) **Abstract**

Provided is an omega-transaminase mutant acquired by a single-point mutation or multi-point mutation at positions 275, 115, and 97 of the amino acid sequence set forth in SEQ ID NO. 2. The transaminase mutant is derived from Aspergillus lentulus. It catalyzes bioreactions with a ketone precursor of a sitagliptin intermediate as the substrate, isopropylamine as the amino donor, pyridoxal phosphate as the coenzyme, and a protonic polar solvent as the cosolvent, thus separating and purifying sitagliptin or the sitagliptin intermediate with high optical purity.

## Description

### TECHNICAL FIELD

The present disclosure relates to an omega-transaminase mutant, an encoding gene, a vector and genetically engineered bacteria containing the encoding gene, and an application thereof in preparation of a sitagliptin intermediate through microbial catalysis.

### BACKGROUND

Sitagliptin is a dipeptidyl peptidase-4 (DPP-4) inhibitor. DPP-4 is a multifunctional enzyme that exists on a cell membrane in the form of a homodimer, which can cleavage a variety of peptide hormones including a glucagon-like peptide-1 and a gastric inhibitory peptide, both of which are closely related to type II diabetes. DPP-4 controls the blood glucose level by protecting endogenous incretin and enhancing its effect. A glucose-dependent insulinotropic peptide (GIP) and the glucagon-like peptide-1 (GLP-1) are incretin released in response to dietary intake.

GLP-1 and GIP can increase insulin synthesis and release from pancreatic beta cells through intracellular signaling pathways, and GLP-1 can also reduce the secretion of glucagon from pancreatic alpha cells, resulting in a decrease in hepatic glucose production. However, both GLP-1 and GIP are rapidly metabolised by DPP-4, resulting in loss of their insulinotropic effect.

The DPP-4 inhibitor inhibits the degradation of incretin DPP-4, thereby enhancing the functions of GLP-1 and GIP, increasing insulin release, and reducing the circulating glucagon level (this effect is glucose-dependent). The DPP-4 inhibitor selectively inhibits DPP-4, but has no inhibitory activity on DPP-8 or DPP-9. In addition, the DPP-4 inhibitor can also inhibit the degradation of GIP, a pituitary adenylate cyclase activated polypeptide, a gastrin-releasing peptide and other peptides that participate in the regulation of blood glucose. Sitagliptin can increase insulin secretion in a blood glucose-dependent manner, and its glucose-lowering effect is moderate, without causing hypoglycemia or side effects such as weight gain, nausea and vomiting. Sitagliptin with the trade name of Januvia was developed by Merck and Codexis in the USA. It is the first dipeptidyl peptidase-IV (DPP-IV) inhibitor approved by the FDA for the treatment of type II diabetes (October 2016). At present, it has been approved in more than 70 countries around the world, and it is reported that the total global sales in 2021 reached 3 billion dollars, and it is one of the top 20 drugs in the international drug sales.

Sitagliptin and an intermediate thereof are synthesised either by a complete chemical method or by a chemical method combined with an enzymatic method. The key of the chemical-enzymatic method is to obtain an omega-transaminase that can catalyse an asymmetric transamination reaction to obtain the optically pure sitagliptin intermediate.

An international patent WO2010099501A2 has disclosed an engineered transaminase developed by Codexis (wild type derived from *Arthrobacter sp.*)*.* It has good tolerance to a solvent DMSO, but has poor tolerance to alcohol solvents. When used in an enzymatic catalytic reaction, DMSO is difficult to remove from a reaction system due to its high boiling point, resulting in greater loss of reaction products in the purification process, and then leading to higher costs.

A U.S. patent US6699871 has disclosed a chemical synthesis method for a sitagliptin intermediate. Chiral alpha-amino acid is induced by a chiral source, which is then diazotised to produce beta-amino acid to construct a required chiral center. The raw material cost required by this route is relatively high, the reaction is more troublesome, and the process and product quality are difficult to control in the industrialization process.

An international patent WO2005003135 has disclosed a synthetic method for synthesising chiral amine by inducing catalytic hydrogenation by S-phenylglycinamide (Merck). Two stages of catalytic hydrogenation are required in this route, a platinum catalyst used in the first stage is expensive, a large amount of PD(OH)₂-C catalyst is required for deprotection in the second stage, the cost is high, and an ee value is 96%, requiring further recrystallization.

An international patent WO2004087650 has disclosed a synthetic route for a sitagliptin intermediate by Merck, in which chiral alcohol is constructed through asymmetric hydrogenation of ketone by a chiral ruthenium catalyst, and then the chiral alcohol is transformed into chiral amine. In this synthetic method, asymmetric hydrogenation of the ruthenium catalyst is required, the catalyst is expensive, the total yield is only 52%, high-pressure hydrogen is used in the process, and the stereoselectivity is also not high.

An international patent WO2007050485 has disclosed a synthetic method for a sitagliptin intermediate by Merck, in which chiral amine is synthesised through asymmetric hydrogenation of enamine by a chiral germanium catalyst, with a yield of 84% and an ee value of 94%. However, this method requires the expensive chiral germanium catalyst which is difficult to remove and recover.

A U.S. patent US8293507 has disclosed that the germanium catalyst in the above process is replaced with a bio-catalyst obtained by transforming a transaminase (ATA117) derived from Arthrobacter by Coexis, and an ee value of a product obtained after transamination reaches 99%.

The following patents have disclosed process routes for a production method for synthesising a sitagliptin intermediate:

A Chinese patent CN107384887 has disclosed that He Renbao et al. screened out a transaminase derived from *Burkholderia gladioli* and carried out engineering transformation on the same. Although the engineered transaminase disclosed in CN107384887 may directly adopt a sitagliptin precursor ketone as a substrate, its activity is not high enough, and it is necessary to use 50 g/L wet cells to achieve a high transformation rate, and DMSO is still used as a solvent in a reaction system. Components of the reaction system become extremely complicated due to the high-concentration wet cells, which is very unfavorable to the reaction post-treatment and product extraction. Meanwhile, the isolation yield of products is also limited by the removal of DMSO.

A Chinese patent CN102838511 has disclosed a production method for a sitagliptin intermediate by Zhejiang Hisoar Pharmaceutical Co., Ltd., in which chiral epichlorohydrin is subjected to nucleophilic substitution with a Grignard reagent, and then cyanide is used to carry out substituted hydrolysis to synthesisee beta-hydroxyl acid. The total yield of this method is only 40%, and its application is limited due to the adoption of the highly toxic cyanide.

A Chinese patent CN103014081 has disclosed that methyl 3-carbo-4-(2,4,5-trifluorophenyl)butanoate is transaminated into methyl (R)-3-amino-4-(2,4,5-trifluorophenyl) butanoate with a transaminase by EnzymeWorks Inc., without disclosing a specific sequence and a cloning method of the transaminase.

In a Chinese patent CN105018440, an engineered transaminase obtained by transforming a transaminase derived from *Mycobacterium vanbaalenii* PYR-1 by Nanjing Boyou Kangyuan Biotech Co., Ltd. is used to synthesisee a relatively simple sitagliptin intermediate: methyl R-3-amino-4-(2,4,5-trifluorophenyl)butanoate. Although the engineered transaminase disclosed in CN105018440 has good tolerance to ethanol and other alcohol solvents (50% ethanol is adopted as the solvent, which is beneficial to purification of a product), it has low catalytic activity, it is necessary to add 10 g/L apoenzymes, the product obtained after a transamination reaction needs to be further transformed into Boc-protected sitagliptin after being protected by Boc, sitagliptin is obtained after deprotection, and the overall yield is not high, leading to high costs.

In recent years, the chemical-enzymatic method has gradually become the first choice for the synthesis of chiral pharmaceutical chemicals and intermediates thereof due to its high selectivity and environment optimization. Omega-transaminases are key enzymes for the production of sitagliptin, genes of many omega-transaminases have been cloned, some of which have been expressed in different hosts (*Escherichia coli*, *Pichia pastoris*, etc.), and genetically engineered bacteria with high enzyme activity and selectivity are obtained. However, there are fewer reports on R-type selective transamination of natural omega-transaminases, and due to the narrow substrate spectra catalysed by these omega-transaminases, and these omega-transaminases are often the most suitable bio-catalysts screened for specific reactions, resulting in greatly limiting their application range. With the development of a directed evolution technology, protein engineering is increasingly used to modify the substrate specificity of enzymes, screen novel transaminases with wide substrate spectra, and study chiral drugs and intermediates thereof that can be efficiently catalysed in high selectivity thereby, which can not only broaden their application range and enhance their application potential, but also lay a foundation for industrial production.

### SUMMARY

The present disclosure aims to provide an engineered transaminase which directly adopts a sitagliptin precursor ketone as a substrate and has better activity, better tolerance to organic solvents and better thermal stability, namely an omega-transaminase mutant, an encoding gene, a vector and genetically engineered bacteria containing the encoding gene, and an application thereof in preparation of a sitagliptin intermediate through microbial catalysis, so as to overcome the defects of an engineered transaminase for synthesising sitagliptin or a chiral amino intermediate thereof in the prior art.

The technical solutions of the present disclosure are as follows:

An omega-transaminase mutant is obtained by carrying out single-point mutation or multi-point combined mutation at positions 275, 115, and 97 of an amino acid sequence shown in SEQ ID NO. 2.

The amino acid sequence shown in SEQ ID NO. 2 is as follows:

MATMDKVFAGYYARQKLLERSDNPFSKGIAYVEGKFVLPSEARIPLLDEGFM
GSDLTYDGFHVWDGRFFRLDDHLQRLLESCDKMRLKFPLALSSVKKILVEMVAKSGI
RDAMGKIIVTRGLTGVQGSKPEDLYNNNIYLLVLPYIWLMAPEKQRHGGSAIITRTVR
RTPPGAFDPTIKNLQWGDLIRGMFEAKDRGATYPFLTDGDTHLTEGPGFNIVLVKNGIL
YTPDRGVLEGITRKSVIEVARANSIDVRLEVVPVETAYHADEIFMCTTGGGIMPITLLD
GKPVNDGQVGPITKKIWDGYWEMHYNAAYSFPVDYGSD

An encoding gene thereof is shown in SEQ ID NO. 1:

ATGGCAACAA TGGATAAAGT GTTTGCAGGT TATTATGCAC
GTCAGAAACT GCTGGAACGT 60

AGCGATAACC CGTTTAGCAA AGGCATCGCA TACGTTGAAG
GTAAATTTGT GCTGCCTAGC 120
GAAGCCCGTA TCCCTCTGCT GGATGAAGGT TTTATGGGCA
GCGATCTGAC CTATGATGGC 180
TTTCATGTGT GGGATGGCCG TTTTTTTCGT CTGGATGATC
ATCTGCAGCG TCTGCTGGAA 240
AGCTGTGATA AAATGCGCCT GAAATTTCCT CTGGCACTGA
GCTCTGTGAA AAAGATCCTG 300
GTGGAAATGG TGGCGAAAAG CGGTATCCGT GATGCAATGG
GTAAAATCAT CGTGACCCGC 360
GGTCTGACAG GTGTGCAGGG TAGCAAACCT GAAGATCTGT
ATAACAACAA CATCTATCTG 420
CTGGTGCTGC CGTATATCTG GCTGATGGCA CCTGAAAAAC
AGCGTCATGG TGGCTCTGCT 480
ATCATCACAC GTACCGTTCG TCGCACACCT CCTGGTGCAT
TTGATCCTAC AATCAAAAAC 540
CTGCAGTGGG GCGATCTGAT CCGTGGTATG TTTGAAGCCA
AAGATCGTGG CGCCACCTAT 600
CCTTTTCTGA CAGATGGTGA TACCCATCTG ACCGAAGGCC
CTGGTTTTAA CATCGTGCTG 660
GTTAAAAACG GTATCCTGTA TACCCCTGAT CGTGGTGTTC
TGGAAGGTAT TACCCGTAAA 720
AGCGTGATCG AAGTGGCGCG TGCAAATAGT ATCGATGTCC
GTCTGGAAGT GGTGCCTGTT 780
GAAACAGCAT ATCATGCCGA TGAAATCTTT ATGTGCACCA
CCGGTGGCGG TATTATGCCT 840
ATTACCCTGC TGGATGGCAA ACCGGTTAAC GATGGTCAGG
TTGGTCCGAT TACCAAAAAG 900
ATCTGGGATG GCTATTGGGA AATGCATTAT AACGCAGCAT
ATAGCTTTCC TGTGGATTAT 960
GGTAGCGAT

Protein consisting of the amino acid sequence shown in SEQ ID NO. 2 may be isolated from *Aspergillus lentulus*, or isolated from an expression transformant that recombinantly expresses the protein, or obtained by artificial synthesis. The identity of the two amino acid sequences or two nucleotide sequences may be obtained through a common algorithm in the art, preferably, calculated through NCBI Blastp and Blastn software according to default parameters.

The mutant includes one or a combination of two or more of the following mutations: (1) mutation of glycine at the position 275 to alanine; (2) mutation of lysine at the position 115 to methionine; and (3) mutation of lysine at the position 97 to arginine.

Preferably, the amino acid sequence of the mutant is shown in SEQ ID No. 4 (mutant G275A), SEQ ID No. 6 (mutant K115M), or SEQ ID No. 8 (mutant K97R/K115M).

Derivative amino acid sequences, which are substituted, deleted or added with one or more amino acid residues and possess transaminase activity, having proteins that have the identity of at least 95% to the amino acid sequence, all belong to the scope of protection of the present disclosure.

The present disclosure further relates to a gene encoding the omega-transaminase mutant.

Specifically, a nucleotide sequence of the encoding gene is shown in SEQ ID NO. 3 (encoding the mutant shown in SEQ ID No. 4) or SEQ ID NO. 5 (encoding the mutant shown in SEQ ID No. 6) or SEQ ID NO. 7 (encoding the mutant shown in SEQ ID No. 8).

Due to the degeneracy of nucleotide codons, polynucleotide sequences encoding the amino acid sequences shown in SEQ ID No. 4, 6, and 8 are not only limited to SEQ ID No. 3, 5, and 7. A nucleic acid sequence encoding the engineered transaminase of the present disclosure may also be any other nucleic acid sequences encoding the amino acid sequences shown in SEQ ID No. 4, 6, and 8 in a sequence table.

The present disclosure further relates to a recombinant vector and genetically engineered bacteria containing the gene encoding the omega-transaminase mutant.

The present disclosure further relates to an application of the omega-transaminase mutant in preparation of sitagliptin or a sitagliptin intermediate through microbial catalysis. Specifically: the recombinant vector containing the transaminase gene is constructed, the shown recombinant vector is transformed into *Escherichia coli,* the obtained recombinant genetically engineered bacteria are subjected to inducing incubation, an incubation broth is isolated to obtain bacterial cells containing the recombinant transaminase, and lysed transaminase crude enzyme solution and purified transaminase pure enzymes are used to prepare sitagliptin or the sitagliptin intermediate. Catalysts include a transaminase and mutant pure enzymes thereof, wet cells of the corresponding recombinant genetically engineered bacteria, crude enzyme solution, crude enzyme powder, pure enzyme solution, pure enzyme powder or the like.

Specifically, the application is as follows: forming a reaction system with a sitagliptin precursor ketone shown in Formula (I) as a reaction substrate, wet cells containing the omega-transaminase mutant as a bio-catalyst, a protonic polar solvent as a cosolvent, pyridoxal phosphate (PLP) as a coenzyme, isopropylamine as a cosubstrate, and a triethanolamine buffer with pH of 8-9 as a reaction medium, carrying out a bio-catalytic reaction at a temperature of 30-50°C and a stirring speed of 100-800 r/min, and isolating and purifying reaction solution after the reaction, to obtain (R)-3-amino-1-piperidine-4-(2,4,5-trifluorophenyl)-1-butanone (II).

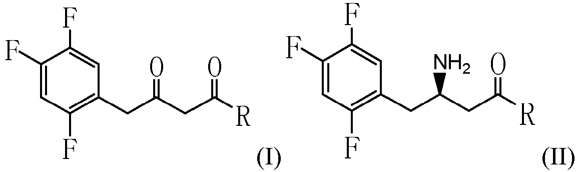

Wherein, in Formula (I) and Formula (II), R is C1-C10 alkyl or alkoxy or piperidyl or morpholinyl or pyrazinyl.

Or, the application is as follows: forming a reaction system with (2Z)-4-oxo-4-[3-(trifluoromethyl)-5,6-dihydro-[1,2,4]triazolo[4,3-a]pyrazine-7(8H)-yl]-1-(2,4 ,5-trifluorophenyl)butan-2-one as a reaction substrate, wet cells containing the omega-transaminase mutant as a bio-catalyst, a protonic polar solvent as a cosolvent, pyridoxal phosphate as a coenzyme, isopropylamine as a cosubstrate, and a triethanolamine buffer with pH of 8-9 as a reaction medium, carrying out a bio-catalytic reaction at a temperature of 30-50°C and a stirring speed of 100-800 r/min, and isolating and purifying reaction solution after the reaction, to obtain sitagliptin.

The protonic polar solvent is preferably selected from one or a mixture of two or more of the following: dimethyl sulfoxide (DMSO), dimethyl formamide (DMF), isopropanol, and ethanol.

In the reaction system, a use amount of the wet cells is 10-50 g/L (preferably, 50 g/L), a final concentration of the substrate is 50-200 g/L, a final volume concentration of the protonic polar solvent is 40-70% (v/v), pyridoxal phosphate is 0.5-2 g/L, and isopropylamine is 5-20 g/L.

The wet cells may be prepared through the following method: inoculating recombinant *Escherichia coli* containing the encoding gene for the omega-transaminase mutant into an LB liquid medium containing 50 µg/ml kanamycin for incubation at 37°C and 200 rpm for 12 hours, inoculating into a fresh LB resistant liquid medium containing 50 µg/ml kanamycin at an inoculum amount of 1% (volume concentration) for incubation at 37°C and 150 rpm till cell OD₆₀₀ reaches 0.6-0.8, adding IPTG at a final concentration of 0.1 mM, centrifuging at 4°C and 5,000 rpm for 20 minutes after inducing incubation at 28°C for 12 hours, discarding a supernatant, and harvesting cell debris, so as to obtain the wet cells.

The present disclosure has the main beneficial effects: as for the problems that reported asymmetric synthesis of sitagliptin and the intermediate thereof has low total yield (generally less than 50%) and low stereoselectivity (an e.e. value of the product is generally less than 90%), a metal catalyst is expensive, and the bio-catalyst cannot directly use the sitagliptin precursor ketone as the substrate, the present disclosure provides a transaminase mutant (bio-catalyst) derived from *Aspergillus lentulus,* the bio-catalytic reaction is carried out with the sitagliptin intermediate precursor ketone (such as 1-piperidine-4-(2,4,5-trifluorophenyl)-1,3-dibutanone) as a substrate, isopropylamine as an amino donor, pyridoxal phosphate as the coenzyme, and the protonic polar solvent as the cosolvent, and then isolation and purification are carried out, to prepare the sitagliptin intermediate or sitagliptin with high optical purity. The method has a total yield of 66.7% (including a transformation yield and a isolation and purification yield) and an e.e. value of the product of 99% (high stereoselectivity), and has good application prospects.

### BRIEF DESCRIPTION OF THE DRAWINGS

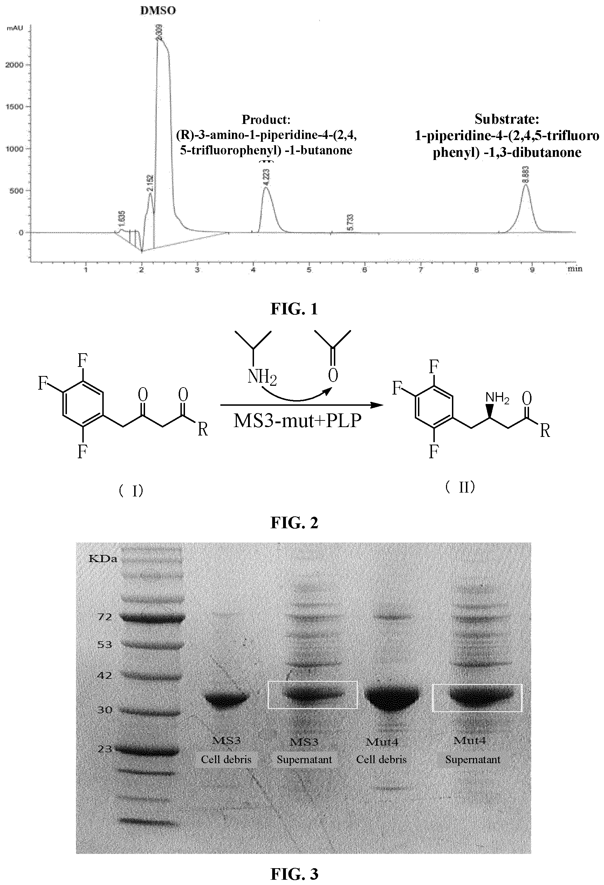

FIG. 1 is a diagram showing HPLC detection results of reaction solution;
FIG. 2 shows reaction formulas of sitagliptin intermediates synthesised through bio-catalysis; and
FIG. 3 shows an electrophoresis detection result of target protein in Embodiment 4.

### DETAILED DESCRIPTION OF THE EMBODIMENTS

The present disclosure will be further described below with reference to specific examples, but the present disclosure is not limited by the following examples.

### Example 1: Amplification of aminotransferase gene MS3

Based on gene sequence information of a transaminase derived from *Aspergillus lentulus* recorded in Genbank, total genome DNA of *Aspergillus lentulus* was extracted with a FastDNA^{®} SPIN Kit, and PCR amplification was carried out under the action of a primer 1 (ATGGGTATCGACACCGGTACCTC) and a primer 2 (GTACTGGATAGCTTCGATCAGCG) with the genome DNA as a template. PCR reaction system (total volume: 50 µL): 25 µL of 10×Pfu DNA Polymerase Buffer, 1 µL of 10 mM dNTP mixture (each of dATP, dCTP, dGTP, and dTTP: 2.5 mM), 1 µL of a cloning primer 1 at a concentration of 50 µM, 1 µL of a cloning primer 2 at a concentration of 50 µM, 1 µL of genome DNA, 1 µL of Pfu DNA Polymerase, and 20 µL of ddH₂O.

A PCR instrument from BioRad was adopted. PCR reaction conditions: predegeneration at 95°C for 5 minutes, degradation at 95°C for 30 seconds, annealing at 65°C for 30 seconds, extension at 72°C for 1 minute for 30 cycles, and finally extension at 72°C for 5 minutes. It can be shown from the results that a length of a nucleotide sequence amplified by the primer 1 and the primer 2 is 936 bp (a nucleotide sequence of an MS3 gene is shown in SEQ ID NO. 1, and an amino acid sequence of an encoded protein is shown in SEQ ID NO. 2), and the sequence encodes a complete open reading frame.

### Example 2: Construction of recombinant Escherichia coli BL21/pET28b-MS3

A primer 3 (ATACCGCCGGCGGTGGTGCACATAAAGA) and a primer 4 (GCACCACCGCCGGCGGTATTATGCCTATA) were designed according to the MS3 gene sequence in Example 1, and PCR amplification was carried out by the primer 3 and the primer 4 under the action of a high-fidelity polymerase (Phanta Max Super-FIDelity DNA Polymerase).

PCR reaction system (total reaction system: 50 µL): 25 µL of 1 ×Phanta max Buffer, 1 µL of 10mM dNTP mixture (each of dATP, dCTP, dGTP, and dTTP: 2.5 mM), 1 µL of Phanta Max Super-FIDelity DNA Polymerase, 1 µL of a cloning primer 3 at a concentration of 50 µM, 1 µL of a cloning primer 4 at a concentration of 50 µM, 1 µL of genome DNA, and 20 µL of nucleic-acid-free water.

A PCR instrument from BioRad was adopted. PCR reaction conditions: predegeneration at 95°C for 5 minutes, degradation at 95°C for 30 seconds, annealing at 58°C for 30 seconds, extension at 72°C for 4 minutes for 30 cycles, and finally extension at 72°C for 5 minutes.

PCR reaction solution was subjected to 0.9% agarose gel electrophoresis detection, and 1 µL of DpnI and 5 µL of CutSmart^{®} Buffer were added to PCR reaction solution 1 and reaction solution 2 respectively for digestion for 2 hours. A target gene was linked to a plasmid vector through a one-step cloning reaction, so as to obtain a recombinant expression vector pET28b-MS3. One-step cloning reaction system: 2 µL of ExnaseTMII, 4 µL of 5×CEIIBuffer, 1 µL of PCR reaction solution 1, 1 µL of PCR reaction solution 2, and 12 µL of nucleic-acid-free water.

10 µL of the above recombinant expression vector pET28b-MS3 was transformed into *Escherichia coli* BL21 (DE3) (Invitrogen) (42°C, 90 seconds), the transformed *Escherichia coli* was spread on an LB plate containing 50 µg/ml kanamycin resistance, and incubated overnight at 37°C, clones were randomly picked to extract plasmids for sequencing identification, and the recombinant *Escherichia coli* BL21 (DE3)/pET28b-MS3 containing recombinant expression plasmids pET28b-MS3 was obtained through screening.

### Example 3: Induction expression of transaminase omega-MS3

The recombinant *Escherichia coli* BL21 (DE3)/pET28b-MS3 obtained in Example 2 was inoculated into an LB liquid medium containing 50 µg/ml kanamycin resistance for incubation at 37°C and 200 rpm for 12 hours and then inoculated into a fresh LB liquid medium containing 50 µg/ml kanamycin resistance at an inoculum amount of 1% (v/v) for incubation at 37°C and 150 rpm till cell OD₆₀₀ reached 0.6-0.8, IPTG at a final concentration of 0.1 mM was added, centrifugation was carried out at 4°C and 5,000 rpm for 25 minutes after inducing incubation was carried out at 28°C for 12 hours, a supernatant was discarded, and cell debris was harvested, so as to obtain wet cells of the recombinant *Escherichia coli* BL21/pET28b-MS3 containing the recombinant expression plasmids. The cells may be directly used as a bio-catalyst or used for protein purification.

### Example 4: Isolation and purification of transaminase omega-MS3

The wet cells obtained in Example 3 were resuspended with a binding buffer (50 mM sodium phosphate buffer with pH of 8.0), ultrasonically crushed (crushing at 240 W under an ice bath condition for 10 minutes, with a duty cycle of 1 second on and 2 seconds off), and centrifuged at 12,000 rpm for 10 minutes, after a supernatant was incubated with Ni affinity chromatography resin that has been equilibrated by the above binding buffer, the Ni affinity chromatography resin was flushed with a flushing buffer (50 mM sodium phosphate buffer with pH of 8.0, containing 300 mM NaCl and 20 mM imidazole) till there was basically no impure protein, then target protein was eluted with an elution buffer (50 mM sodium phosphate buffer with pH of 8.0, containing 300 mM NaCl and 500 mM imidazole) and harvested, the target protein was merged after purity was identified through electrophoresis, and the merged target protein was dialyzed with a dialysis buffer (50 mM sodium phosphate buffer with pH of 8.0) for 48 hours (molecular weight cut-off of a dialysis bag: 33 KDa). The protein content was determined as 1.8 mg/mL through a Coomassie brilliant blue method, enzyme solution was diluted to a final concentration of 0.5 mg/mL with the 50 mM sodium phosphate with pH of 8.0, and subpackaged and cryopreserved at -80°C.

### Example 5: Construction of mutant strain library

According to a parental transaminase basic sequence derived from *dergillus lentulus* recorded in Genbank (the sequence is shown in SEQ ID NO. 2, and a nucleotide sequence is shown in SEQ ID NO. 1), mutant primers for site-directed mutation were designed, single-point mutation or multi-point mutation was introduced to positions 275, 115, and 97 with a recombinant vector pET28b-MS3 as a template, and the primers were as follows:
primer 5: G275A Pf ATACCGCCGGCGGTGGTGCACATAAAGA
G275APr GCACCACCGCCGGCGGTATTATGCCTATA
primer 6: K115M Pf ACGATGATCATACCCATTGCATCACGGATA
K115M Pr TTGTTGCCATGGTATATCTCCTTCTTAAAGTT
primer 7: K97R Pf AGCTCTGTGCGCAAGATCCTGGTGGAAATGG
K97R Pr CCAGGATCTTGCGCACAGAGCTCAGTGCC

PCR reaction system (total reaction system: 50 µL): 25 µL of 1×Phanta max Buffer, 1 µL of 10 mM dNTP mixture (each of dATP, dCTP, dGTP, and dTTP: 2.5 mM), 1 µL of Phanta Max Super-FIDelity DNA Polymerase, 1 µL of a forward primer at a concentration of 50 µM, 1 µL of a reverse primer at a concentration of 50 µM, 1 µL of the recombinant vector pET28b-MS3, and 20 µL of nucleic-acid-free water.

PCR reaction conditions: predegeneration at 95°C for 5 minutes, degradation at 95°C for 30 seconds, annealing at 65°C for 30 seconds, extension at 72°C for 6 minutes for 30 cycles, and finally extension at 72°C for 5 minutes.

10 µL of a PCR product was transfected into competent cells containing 100 µL of *Escherichia coli* BL21 (DE3) for thermal shock for 90 seconds under a transformation condition of 42°C and rapidly put on ice to be cooled for 3 minutes, 600 µL of an LB liquid medium was added to a tube for incubation at 37°C and 180 r/min for 60 minutes, and centrifuged at 12,000 rpm for 1 minute, 600 µL of a supernatant was discarded, 100 µL of a remaining bacterial solution was fully mixed well and spread on an LB resistant plate containing 50 µg/ml ampicillin, and after the bacterial solution was completely absorbed by the medium, inverted incubation was carried out at 37°C for 14-16 hours.

A monoclonal strain was picked from the LB resistant plate containing 50 µg/ml ampicillin and sent to Tsingke for sequence detection, and a sequencing result was analysed through software.

### Example 6: Determination of enzyme activity of mutant

After *Escherichia coli* containing mutant protein was obtained, a sitagliptin intermediate precursor ketone at a final concentration of 25 g/L was bio-transformed to be screened. Final concentration composition and catalytic conditions of a catalytic system (1 ml): 15 g/L triethanolamine, triethanolamine buffer with pH of 8.5-9.0, 25 g/L sitagliptin intermediate precursor ketone 1-piperidine-4-(2,4,5-trifluorophenyl)-1,3-dibutanone as a substrate, DMSO at a final concentration of 50% (v/v), 0.5 g/L pyridoxal phosphate, and 40 g/L isopropylamine.

Reaction conditions: temperature of 45°C, stirring speed of 600 r/min, and reaction time of 12 hours. Under the same conditions, reaction solution to which unmutated wet cells were added was used as a blank control. Samples were taken for HPLC detection after the reaction was completed.

HPLC detection conditions: Mobile phase A: 10 mM ammonium acetate; mobile phase B: pure acetonitrile; mobile phase A: mobile phase B = 1:1; flow rate: 1 ml/min; and detection wavelength: 205 nm. The enzyme activity of the mutant is shown in Table 1.

**Table 1**

| | Mutation site | Final concentration of substrate | Specific enzyme activity (U/g) |
|---|---|---|---|
| BL21 (DE3) /pET28b-MS3 | WT | 25g/L | 11.3 |
| BL21/pET28b-MS3mut1 | G275A | 25g/L | 28.8 |
| BL21/pET28b-MS3mut2 | K115M | 25g/L | 23.8 |
| BL21/pET28b-MS3mut3 | K97R | 25g/L | 29.5 |
| BL21/pET28b-MS3mut4 | K97R/K115M | 25g/L | 66.8 |
| BL21/pET28b-MS3mut5 | G275A/K115M | 25g/L | 20.9 |
| BL21/pET28b-MS3mut6 | G275A/K97R | 25g/L | 21.7 |
| BL21/pET28b-MS3mut7 | G275A/K115M/K97R | 25g/L | 22.0 |

### Example 7: Application of recombinant transaminase MS3 in preparation of sitagliptin intermediate (R)-3-amino-1-piperidine-4-(2,4,5-trifluorophenyl)-1-butanone

The sitagliptin intermediate (R)-3-amino-1-piperidine-4-(2,4,5-trifluorophenyl)-1-butanone was synthesised through a bio-catalytic reaction carried out with the recombinant *Escherichia coli* BL21/pET28b-MS3 wet cells containing the recombinant expression plasmids obtained by the method in Example 3 as a bio-catalyst and the sitagliptin intermediate precursor ketone [1-piperidine-4-(2,4,5-trifluorophenyl)-1,3-dibutanone] as a substrate.

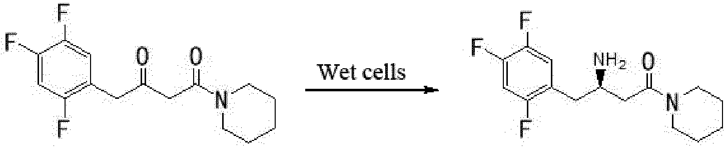

Final concentration composition and catalytic conditions of a catalytic system at a low substrate concentration (1 ml): 0.25 g of wet cells, triethanolamine buffer with pH of 8-8.5, 5 g/L substrate sitagliptin precursor ketone, DMSO at a final concentration of 50% (v/v), 0.5 g/L pyridoxal phosphate, and 10 g/L isopropylamine. Reaction conditions: temperature of 45°C, stirring speed of 1,200 r/min, and reaction time of 36 hours. Under the same conditions, reaction solution without cells added was used as a blank control, and the *Escherichia coli* BL21/pET28b wet cells replaced the above recombinant *Escherichia coli* BL21/pET28b-MS3 as a negative control. Samples were taken for HPLC detection after the reaction was completed, a transformation rate of the substrate of the reaction system was 16.9%, and an ee value was greater than 99%.

Final concentration composition and catalytic conditions of a catalytic system at a high substrate concentration (1 ml): 0.25 g of wet cells, triethanolamine buffer with pH of 8-8.5, 50 g/L substrate sitagliptin precursor ketone, DMSO at a final concentration of 50% (v/v), 0.5 g/L pyridoxal phosphate, and 10 g/L isopropylamine. Reaction conditions: temperature of 45°C, stirring speed of 1200 r/min, and reaction time of 36 hours. Under the same conditions, the reaction solution without cells added was used as the blank control, and the *Escherichia coli* BL21/pET28b wet cells replaced the above recombinant *Escherichia coli* BL21/pET28b-MS3 as the negative control. Samples were taken for HPLC detection (conditions were the same as those in Example 5) after the reaction was completed, and the transformation rate of the substrate of the reaction system was less than 10% (9.5%).

### Example 8: Application of recombinant transaminase MS3 mutant 1 in preparation of sitagliptin intermediate (R)-3-amino-1-piperidine-4-(2,4,5-trifluorophenyl)-1-butanone

The sitagliptin intermediate (R)-3-amino-1-piperidine-4-(2,4,5-trifluorophenyl)-1-butanone was synthesised through a bio-catalytic reaction carried out with the recombinant *Escherichia coli* BL21/pET28b-MS3mut1 wet cells containing the recombinant expression plasmids obtained by the method in Example 3 as a bio-catalyst and the sitagliptin intermediate precursor ketone [1-piperidine-4-(2,4,5-trifluorophenyl)-1,3-dibutanone] as a substrate.

Final concentration composition and catalytic conditions of a catalytic system at a low substrate concentration (1 ml): 0.25 g of wet cells, triethanolamine buffer with pH of 8-8.5, 2 g/L sitagliptin precursor ketone as the substrate, DMSO at a final concentration of 50% (v/v), 0.5 g/L pyridoxal phosphate, and 10 g/L isopropylamine. Reaction conditions: temperature of 45°C, stirring speed of 1,200 r/min, and reaction time of 36 hours. Under the same conditions, reaction solution without cells added was used as a blank control, and the *Escherichia coli* BL21/pET28b wet cells replaced the above recombinant *Escherichia coli* BL21/pET28b-MS3mut1 as a negative control. Samples were taken for HPLC detection after the reaction was completed. At this concentration, a transformation rate of the MS3 mutant 1 for the substrate was 66.7%, and an ee value was greater than 99%.

Final concentration composition and catalytic conditions of a catalytic system at a high substrate concentration (1ml): 0.75 g of wet cells, triethanolamine buffer with pH of 8-8.5, 50 g/L sitagliptin precursor ketone as the substrate, DMSO at a final concentration of 50% (v/v), 0.5 g/L pyridoxal phosphate, and 10 g/L isopropylamine. Reaction conditions: temperature of 45°C, stirring speed of 1200 r/min, and reaction time of 36 hours. Under the same conditions, the reaction solution without cells added was used as the blank control, and the *Escherichia coli* BL21/pET28b wet cells replaced the above recombinant *Escherichia coli* BL21/pET28b-MS3mut1 as the negative control. Samples were taken for HPLC detection after the reaction was completed. At this concentration, a transformation rate of the MS3 mutant 1 for the substrate was 56.0%, and an ee value was greater than 99%.

### Example 9: Application of recombinant transaminase MS3 mutant 2 in preparation of sitagliptin intermediate (R)-3-amino-1-piperidine-4-(2,4,5-trifluorophenyl)-1-butanone

The sitagliptin intermediate (R)-3-amino-1-piperidine-4-(2,4,5-trifluorophenyl)-1-butanone was synthesised through a bio-catalytic reaction carried out with the recombinant *Escherichia coli* BL21/pET28b-MS3mut2 wet cells containing the recombinant expression plasmids obtained by the method in Example 3 as a bio-catalyst and the sitagliptin intermediate precursor ketone [1-piperidine-4-(2,4,5-trifluorophenyl)-1,3-dibutanone] as a substrate.

Final concentration composition and catalytic conditions of a catalytic system at a low substrate concentration (1 ml): 0.25 g of wet cells, triethanolamine buffer with pH of 8-8.5, 5 g/L sitagliptin precursor ketone as the substrate, DMSO at a final concentration of 50% (v/v), 0.5 g/L pyridoxal phosphate, and 10 g/L isopropylamine. Reaction conditions: temperature of 45°C, stirring speed of 1,200 r/min, and reaction time of 36 hours. Under the same conditions, reaction solution without cells added was used as a blank control, and the *Escherichia coli* BL21/pET28b wet cells replaced the above recombinant *Escherichia coli* BL21/pET28b-MS3mut2 as a negative control. Samples were taken for HPLC detection after the reaction was completed. At this concentration, a transformation rate of the MS3 mutant 2 for the substrate was 74.7%, and an ee value was greater than 99%.

Final concentration composition and catalytic conditions of a catalytic system at a high substrate concentration (1 ml): 0.75 g of wet cells, triethanolamine buffer with pH of 8-8.5, 50 g/L sitagliptin precursor ketone as the substrate, DMSO at a final concentration of 50% (v/v), 0.5 g/L pyridoxal phosphate, and 10 g/L isopropylamine. Reaction conditions: temperature of 45°C, stirring speed of 1200 r/min, and reaction time of 36 hours. Under the same conditions, reaction solution without cells added was used as a blank control, and the *Escherichia coli* BL21/pET28b wet cells replaced the above recombinant *Escherichia coli* BL21/pET28b-MS3mut2 as a negative control. Samples were taken for HPLC detection after the reaction was completed. At this concentration, a transformation rate of the MS3 mutant 2 for the substrate was 68.1%, and an ee value was greater than 99%.

### Example 10: Application of recombinant transaminase MS3 mutant 3 in preparation of sitagliptin intermediate (R)-3-amino-1-piperidine-4-(2,4,5-trifluorophenyl)-1-butanone

The sitagliptin intermediate (R)-3-amino-1-piperidine-4-(2,4,5-trifluorophenyl)-1-butanone was synthesised through a bio-catalytic reaction carried out with the recombinant *Escherichia coli* BL21/pET28b-MS3mut3 wet cells containing the recombinant expression plasmids obtained by the method in Example 3 as a bio-catalyst and the sitagliptin intermediate precursor ketone [1-piperidine-4-(2,4,5-trifluorophenyl)-1,3-dibutanone] as a substrate.

Final concentration composition and catalytic conditions of a catalytic system at a low substrate concentration (1 ml): 0.25 g of wet cells, triethanolamine buffer with pH of 8-8.5, 5 g/L sitagliptin precursor ketone as the substrate, DMSO at a final concentration of 50% (v/v), 0.5 g/L pyridoxal phosphate, and 10 g/L isopropylamine. Reaction conditions: temperature of 45°C, stirring speed of 1,200 r/min, and reaction time of 36 hours. Under the same conditions, reaction solution without cells added was used as a blank control, and the *Escherichia coli* BL21/pET28b wet cells replaced the above recombinant *Escherichia coli* BL21/pET28b-MS3mut3 as a negative control. Samples were taken for HPLC detection after the reaction was completed. At this concentration, a transformation rate of the MS3 mutant 3 for the substrate was 87.8%, and an ee value was greater than 99%.

Final concentration composition and catalytic conditions of a catalytic system at a high substrate concentration (1 ml): 0.75 g of wet cells, triethanolamine buffer with pH of 8-8.5, 50 g/L sitagliptin precursor ketone as the substrate, DMSO at a final concentration of 50% (v/v), 0.5 g/L pyridoxal phosphate, and 10 g/L isopropylamine. Reaction conditions: temperature of 45°C, stirring speed of 1200 r/min, and reaction time of 36 hours. Under the same conditions, reaction solution without cells added was used as a blank control, and the *Escherichia coli* BL21/pET28b wet cells replaced the above recombinant *Escherichia coli* BL21/pET28b-MS3mut3 as a negative control. Samples were taken for HPLC detection after the reaction was completed. At this concentration, a transformation rate of the MS3 mutant 3 for the substrate was 83.9%, and an ee value was greater than 99%.

### Example 11: Application of recombinant transaminase MS3 mutant 4 in preparation of sitagliptin intermediate (R)-3-amino-1-piperidine-4-(2,4,5-trifluorophenyl)-1-butanone

The sitagliptin intermediate (R)-3-amino-1-piperidine-4-(2,4,5-trifluorophenyl)-1-butanone was synthesised through a bio-catalytic reaction carried out with the recombinant *Escherichia coli* BL21/pET28b-MS3mut4 wet cells containing the recombinant expression plasmids obtained by the method in Example 3 as a bio-catalyst and the sitagliptin intermediate precursor ketone [1-piperidine-4-(2,4,5-trifluorophenyl)-1,3-dibutanone] as a substrate.

Final concentration composition and catalytic conditions of a catalytic system at a low substrate concentration (1 ml): 0.25 g of wet cells, triethanolamine buffer with pH of 8-8.5, 5 g/L sitagliptin precursor ketone as the substrate, DMSO at a final concentration of 50% (v/v), 0.5 g/L pyridoxal phosphate, and 10 g/L isopropylamine. Reaction conditions: temperature of 45°C, stirring speed of 1,200 r/min, and reaction time of 36 hours. Under the same conditions, reaction solution without cells added was used as a blank control, and the *Escherichia coli* BL21/pET28b wet cells replaced the above recombinant *Escherichia coli* BL21/pET28b-MS3mut4 as a negative control. Samples were taken for HPLC detection after the reaction was completed. At this concentration, a transformation rate of the MS3 mutant 4 for the substrate was 95.2%, and an ee value was greater than 99%.

Final concentration composition and catalytic conditions of a catalytic system at a high substrate concentration (1 ml): 0.75 g of wet cells, triethanolamine buffer with pH of 8-8.5, 50 g/L sitagliptin precursor ketone as the substrate, DMSO at a final concentration of 50% (v/v), 0.5 g/L pyridoxal phosphate, and 10 g/L isopropylamine. Reaction conditions: temperature of 45°C, stirring speed of 1200 r/min, and reaction time of 36 hours. Under the same conditions, reaction solution without cells added was used as a blank control, and the *Escherichia coli* BL21/pET28b wet cells replaced the above recombinant *Escherichia coli* BL21/pET28b-MS3mut4 as a negative control. Samples were taken for HPLC detection after the reaction was completed. At this concentration, a transformation rate of the MS3 mutant 4 for the substrate was 91.7%, and an ee value was greater than 99%.

In conclusion, transformation rates of the other three mutants were subjected to a determination experiment through the method in this example, and results of HPLC detection after the reaction for 36 hours are shown in Table 2:

**Table 2: Transformation rates of different transaminase mutants for substrates at different concentrations and ee values of products**

| Mutant | Mutation site | Transformation rate (%) Substrate concentration 5 g/L | Transformation rate (%) Substrate concentration 50 g/L | *e.e.* (%) |
|---|---|---|---|---|
| MS3 (Comparative Example) | No | 16.9% | 9.5% | >50% |
| Mutant 1 | G275A | 66.7% | 56.0% | >99% |
| Mutant 2 | K115M | 74.7% | 68.1% | >99% |
| Mutant 3 | K97R | 87.8% | 83.9% | >99% |
| Mutant 4 | K97R/K115M | 95.2% | 91.7% | >99% |
| Mutant 5 | G275A/K115M | 67.8% | 59.5% | >99% |
| Mutant 6 | G275A/K97R | 68.9% | 59.9% | >99% |
| Mutant 7 | G275A/K115M/K97R | 69.3% | 61.6% | >99% |

### Example 12: Application of recombinant transaminase MS3 mutant 5 in preparation of sitagliptin intermediate (R)-3-amino-1-piperidine-4-(2,4,5-trifluorophenyl)-1-butanone

The sitagliptin intermediate (R)-3-amino-1-piperidine-4-(2,4,5-trifluorophenyl)-1-butanone was synthesised through a bio-catalytic reaction carried out with the recombinant *Escherichia coli* BL21/pET28b-MS3mut5 wet cells containing the recombinant expression plasmids obtained by the method in Example 3 as a bio-catalyst and the sitagliptin intermediate precursor ketone [1-piperidine-4-(2,4,5-trifluorophenyl)-1,3-dibutanone] as a substrate.

Final concentration composition and catalytic conditions of a catalytic system at a low substrate concentration (1 ml): 0.25 g of wet cells, triethanolamine buffer with pH of 8-8.5, 5 g/L sitagliptin precursor ketone as the substrate, DMSO at a final concentration of 50% (v/v), 0.5 g/L pyridoxal phosphate, and 10 g/L isopropylamine. Reaction conditions: temperature of 45°C, stirring speed of 1,200 r/min, and reaction time of 36 hours. Under the same conditions, reaction solution without cells added was used as a blank control, and the *Escherichia coli* BL21/pET28b wet cells replaced the above recombinant *Escherichia coli* BL21/pET28b-MS3mut5 as a negative control. Samples were taken for HPLC detection after the reaction was completed. At this concentration, a transformation rate of the MS3 mutant 5 for the substrate was 67.8%, and an ee value was greater than 99%.

Final concentration composition and catalytic conditions of a catalytic system at a high substrate concentration (1 ml): 0.75 g of wet cells, triethanolamine buffer with pH of 8-8.5, 50 g/L sitagliptin precursor ketone as the substrate, DMSO at a final concentration of 50% (v/v), 0.5 g/L pyridoxal phosphate, and 10 g/L isopropylamine. Reaction conditions: temperature of 45°C, stirring speed of 1200 r/min, and reaction time of 36 hours. Under the same conditions, reaction solution without cells added was used as a blank control, and the *Escherichia coli* BL21/pET28b wet cells replaced the above recombinant *Escherichia coli* BL21/pET28b-MS3mut5 as a negative control. Samples were taken for HPLC detection after the reaction was completed. At this concentration, a transformation rate of the MS3 mutant 5 for the substrate was 59.5%, and an ee value was greater than 99%.

### Example 13: Application of recombinant transaminase MS3 mutant 6 in preparation of sitagliptin intermediate (R)-3-amino-1-piperidine-4-(2,4,5-trifluorophenyl)-1-butanone

The sitagliptin intermediate (R)-3-amino-1-piperidine-4-(2,4,5-trifluorophenyl)-1-butanone was synthesised through a bio-catalytic reaction carried out with the recombinant *Escherichia coli* BL21/pET28b-MS3mut6 wet cells containing the recombinant expression plasmids obtained by the method in Example 3 as a bio-catalyst and the sitagliptin intermediate precursor ketone [1-piperidine-4-(2,4,5-trifluorophenyl)-1,3-dibutanone] as a substrate.

Final concentration composition and catalytic conditions of a catalytic system at a low substrate concentration (1 ml): 0.25 g of wet cells, triethanolamine buffer with pH of 8-8.5, 5 g/L sitagliptin precursor ketone as the substrate, DMSO at a final concentration of 50% (v/v), 0.5 g/L pyridoxal phosphate, and 10 g/L isopropylamine. Reaction conditions: temperature of 45°C, stirring speed of 1,200 r/min, and reaction time of 36 hours. Under the same conditions, reaction solution without cells added was used as a blank control, and the *Escherichia coli* BL21/pET28b wet cells replaced the above recombinant *Escherichia coli* BL21/pET28b-MS3mut6 as a negative control. Samples were taken for HPLC detection after the reaction was completed. At this concentration, a transformation rate of the MS3 mutant 6 for the substrate was 68.9%, and an ee value was greater than 99%.

Final concentration composition and catalytic conditions of a catalytic system at a high substrate concentration (1 ml): 0.75 g of wet cells, triethanolamine buffer with pH of 8-8.5, 50 g/L sitagliptin precursor ketone as the substrate, DMSO at a final concentration of 50% (v/v), 0.5 g/L pyridoxal phosphate, and 10 g/L isopropylamine. Reaction conditions: temperature of 45°C, stirring speed of 1200 r/min, and reaction time of 36 hours. Under the same conditions, reaction solution without cells added was used as a blank control, and the *Escherichia coli* BL21/pET28b wet cells replaced the above recombinant *Escherichia coli* BL21/pET28b-MS3mut6 as a negative control. Samples were taken for HPLC detection after the reaction was completed. At this concentration, a transformation rate of the MS3 mutant 6 for the substrate was 59.9%, and an ee value was greater than 99%.

### Example 14: Application of recombinant transaminase MS3 mutant 7 in preparation of sitagliptin intermediate (R)-3-amino-1-piperidine-4-(2,4,5-trifluorophenyl)-1-butanone

The sitagliptin intermediate (R)-3-amino-1-piperidine-4-(2,4,5-trifluorophenyl)-1-butanone was synthesised through a bio-catalytic reaction carried out with the recombinant *Escherichia coli* BL21/pET28b-MS3mut7 wet cells containing the recombinant expression plasmids obtained by the method in Example 3 as a bio-catalyst and the sitagliptin intermediate precursor ketone [1-piperidine-4-(2,4,5-trifluorophenyl)-1,3-dibutanone] as a substrate.

Final concentration composition and catalytic conditions of a catalytic system at a low substrate concentration (1 ml): 0.25 g of wet cells, triethanolamine buffer with pH of 8-8.5, 5 g/L sitagliptin precursor ketone as the substrate, DMSO at a final concentration of 50% (v/v), 0.5 g/L pyridoxal phosphate, and 10 g/L isopropylamine. Reaction conditions: temperature of 45°C, stirring speed of 1,200 r/min, and reaction time of 36 hours. Under the same conditions, reaction solution without cells added was used as a blank control, and the *Escherichia coli* BL21/pET28b wet cells replaced the above recombinant *Escherichia coli* BL21/pET28b-MS3mut7 as a negative control. Samples were taken for HPLC detection after the reaction was completed. At this concentration, a transformation rate of the MS3 mutant 7 for the substrate was 69.3%, and an ee value was greater than 99%.

Final concentration composition and catalytic conditions of a catalytic system at a high substrate concentration (1 ml): 0.75 g of wet cells, triethanolamine buffer with pH of 8-8.5, 50 g/L sitagliptin precursor ketone as the substrate, DMSO at a final concentration of 50% (v/v), 0.5 g/L pyridoxal phosphate, and 10 g/L isopropylamine. Reaction conditions: temperature of 45°C, stirring speed of 1200 r/min, and reaction time of 36 hours. Under the same conditions, reaction solution without cells added was used as a blank control, and the *Escherichia coli* BL21/pET28b wet cells replaced the above recombinant *Escherichia coli* BL21/pET28b-MS3mut7 as a negative control. Samples were taken for HPLC detection after the reaction was completed. At this concentration, a transformation rate of the MS3 mutant 7 for the substrate was 61.6%, and an ee value was greater than 99%.

### Example 15: Application of 1-methoxy-4-(2,4,5-trifluorophenyl)-1,3-butanedione as a substrate in synthesis of (R)-3-amino-1-methoxy-4-(2,4,5-trifluorophenyl)-1-butanone

The sitagliptin intermediate (R)-3-amino-1-methoxy-4-(2,4,5-trifluorophenyl)-1-butanone was synthesised through a bio-catalytic reaction carried out with the recombinant *Escherichia coli* BL21/pET28b-MS3mutl wet cells containing the recombinant expression plasmids obtained by the method in Example 3 as a bio-catalyst and the sitagliptin intermediate precursor ketone [1-methoxy-4-(2,4,5-trifluorophenyl)-1,3-butanedione] as a substrate.

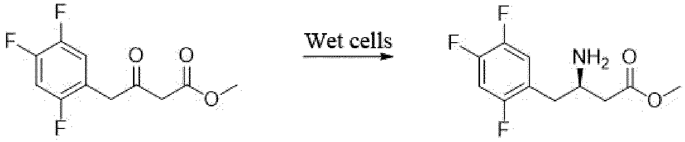

Final concentration composition and catalytic conditions of a catalytic system (100 ml): 25.0 g of wet cells, triethanolamine buffer with pH of 8-8.5, 50 g/L 1-methoxy-4-(2,4,5-trifluorophenyl)-1,3-butanedione as the substrate, DMSO at a final concentration of 50% (v/v), 0.5 g/L pyridoxal phosphate, and 10 g/L isopropylamine. Reaction conditions: temperature of 45°C, stirring speed of 1,200 r/min, and reaction time of 24 hours. Under the same conditions, reaction solution without cells added was used as a blank control, and the *Escherichia coli* BL21/pET28b wet cells replaced the above recombinant *Escherichia coli* BL21/pET28b-MS3 as a negative control. Samples were taken for HPLC detection after the reaction was completed. A transformation rate of the MS3 mutant 1 for the substrate was 94.7%, and an ee value was greater than 99%.

### Example 16: Application of 1-methoxy-4-(2,4,5-trifluorophenyl)-1,3-butanedione as a substrate in synthesis of (R)-3-amino-1-methoxy-4-(2,4,5-trifluorophenyl)-1-butanone

The sitagliptin intermediate (R)-3-amino-1-methoxy-4-(2,4,5-trifluorophenyl)-1-butanone was synthesised through a bio-catalytic reaction carried out with the recombinant *Escherichia coli* BL21/pET28b-MS3mutl wet cells containing the recombinant expression plasmids obtained by the method in Example 3 as a bio-catalyst and the sitagliptin intermediate precursor ketone [1-methoxy-4-(2,4,5-trifluorophenyl)-1,3-butanedione] as a substrate.

Final concentration composition and catalytic conditions of a catalytic system (100 ml): 25.0 g of wet cells, 50 g/L 1-methoxy-4-(2,4,5-trifluorophenyl)-1,3-butanedione as the substrate, isopropanol at a final concentration of 65% (v/v), 0.5 g/L pyridoxal phosphate, and 10 g/L isopropylamine. Reaction conditions: temperature of 45°C, stirring speed of 1,200 r/min, and reaction time of 24 hours. Under the same conditions, reaction solution without cells added was used as a blank control, and the *Escherichia coli* BL21/pET28b wet cells replaced the above recombinant *Escherichia coli* BL21/pET28b-MS3 as a negative control. Samples were taken for HPLC detection after the reaction was completed. A transformation rate of the MS3 mutant 1 for the substrate was 92.5%, and an ee value was greater than 99%.

### Example 17: Application of 1-methoxy-4-(2,4,5-trifluorophenyl)-1,3-butanedione as a substrate in synthesis of (R)-3-amino-1-methoxy-4-(2,4,5-trifluorophenyl)-1-butanone

The sitagliptin intermediate (R)-3-amino-1-methoxy-4-(2,4,5-trifluorophenyl)-1-butanone was synthesised through a bio-catalytic reaction carried out with the recombinant *Escherichia coli* BL21/pET28b-MS3mutl wet cells containing the recombinant expression plasmids obtained by the method in Example 3 as a bio-catalyst and the sitagliptin intermediate precursor ketone [1-methoxy-4-(2,4,5-trifluorophenyl)-1,3-butanedione] as a substrate.

Final concentration composition and catalytic conditions of a catalytic system (100 ml): 25.0 g of wet cells, 50 g/L 1-methoxy-4-(2,4,5-trifluorophenyl)-1,3-butanedione as the substrate, ethanol at a final concentration of 65% (v/v), 0.5 g/L pyridoxal phosphate, and 10 g/L isopropylamine. Reaction conditions: temperature of 45°C, stirring speed of 1,200 r/min, and reaction time of 24 hours. Under the same conditions, reaction solution without cells added was used as a blank control, and the *Escherichia coli* BL21/pET28b wet cells replaced the above recombinant *Escherichia coli* BL21/pET28b-MS3 as a negative control. Samples were taken for HPLC detection after the reaction was completed. A transformation rate of the MS3 mutant 1 for the substrate was 90.6%, and an ee value was greater than 99%.

### Example 18: Application of 1-methoxy-4-(2,4,5-trifluorophenyl)-1,3-butanedione as a substrate in synthesis of (R)-3-amino-1-methoxy-4-(2,4,5-trifluorophenyl)-1-butanone

The sitagliptin intermediate (R)-3-amino-1-methoxy-4-(2,4,5-trifluorophenyl)-1-butanone was synthesised through a bio-catalytic reaction carried out with the recombinant *Escherichia coli* BL21/pET28b-MS3mutl wet cells containing the recombinant expression plasmids obtained by the method in Example 3 as a bio-catalyst and the sitagliptin intermediate precursor ketone [1-methoxy-4-(2,4,5-trifluorophenyl)-1,3-butanedione] as a substrate.

Final concentration composition and catalytic conditions of a catalytic system (100 ml): 25.0 g of wet cells, 50 g/L 1-methoxy-4-(2,4,5-trifluorophenyl)-1,3-butanedione as the substrate, DMF at a final concentration of 50% (v/v), 0.5 g/L pyridoxal phosphate, and 10 g/L isopropylamine. Reaction conditions: temperature of 45°C, stirring speed of 1,200 r/min, and reaction time of 24 hours. Under the same conditions, reaction solution without cells added was used as a blank control, and the *Escherichia coli* BL21/pET28b wet cells replaced the above recombinant *Escherichia coli* BL21/pET28b-MS3 as a negative control. Samples were taken for HPLC detection after the reaction was completed. A transformation rate of the MS3 mutant 1 for the substrate was 91.5%, and an ee value was greater than 99%.

### Example 19: Application of 1-morpholino-4-(2,4,5-trifluorophenyl)-1,3-butanedione as a substrate in synthesis of (R)-3-amino-1-morpholino-4-(2,4,5-trifluorophenyl)-1-butanone

The sitagliptin intermediate (R)-3-amino-1-morpholino-4-(2,4,5-trifluorophenyl)-1-butanone was synthesised through a bio-catalytic reaction carried out with the recombinant *Escherichia coli* BL21/pET28b-MS3mutl wet cells containing the recombinant expression plasmids obtained by the method in Example 3 as a bio-catalyst and the sitagliptin intermediate precursor ketone [1-morpholino-4-(2,4,5-trifluorophenyl)-1,3-butanedione] as the substrate.

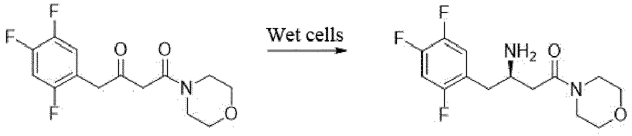

Final concentration composition and catalytic conditions of a catalytic system (100 ml): 25.0 g of wet cells, triethanolamine buffer with pH of 8-8.5, 50 g/L 1-morpholino-4-(2,4,5-trifluorophenyl)-1,3-butanedione as the substrate, DMSO at a final concentration of 50% (v/v), 0.5 g/L pyridoxal phosphate, and 10 g/L isopropylamine. Reaction conditions: temperature of 45°C, stirring speed of 1,200 r/min, and reaction time of 24 hours. Under the same conditions, reaction solution without cells added was used as a blank control, and the *Escherichia coli* BL21/pET28b wet cells replaced the above recombinant *Escherichia coli* BL21/pET28b-MS3 as a negative control. Samples were taken for HPLC detection after the reaction was completed. A transformation rate of the MS3 mutant 1 for the substrate was 95.5%, and an ee value was greater than 99%.

### Example 20: Application of 1-morpholino-4-(2,4,5-trifluorophenyl)-1,3-butanedione as a substrate in synthesis of (R)-3-amino-1-morpholino-4-(2,4,5-trifluorophenyl)-1-butanone

The sitagliptin intermediate (R)-3-amino-1-morpholino-4-(2,4,5-trifluorophenyl)-1-butanone was synthesised through a bio-catalytic reaction carried out with the recombinant *Escherichia coli* BL21/pET28b-MS3mutl wet cells containing the recombinant expression plasmids obtained by the method in Example 3 as a bio-catalyst and the sitagliptin intermediate precursor ketone [1-morpholino-4-(2,4,5-trifluorophenyl)-1,3-butanedione] as the substrate.

Final concentration composition and catalytic conditions of a catalytic system (100 ml): 25.0 g of wet cells, 50 g/L 1-morpholino-4-(2,4,5-trifluorophenyl)-1,3-butanedione as the substrate, isopropanol at a final concentration of 65% (v/v), 0.5 g/L pyridoxal phosphate, and 10 g/L isopropylamine. Reaction conditions: temperature of 45°C, stirring speed of 1,200 r/min, and reaction time of 24 hours. Under the same conditions, reaction solution without cells added was used as a blank control, and the *Escherichia coli* BL21/pET28b wet cells replaced the above recombinant *Escherichia coli* BL21/pET28b-MS3 as a negative control. Samples were taken for HPLC detection after the reaction was completed. A transformation rate of the MS3 mutant 1 for the substrate was 96.2%, and an ee value was greater than 99%.

### Example 21: Application of 1-morpholino-4-(2,4,5-trifluorophenyl)-1,3-butanedione as a substrate in synthesis of (R)-3-amino-1-morpholino-4-(2,4,5-trifluorophenyl)-1-butanone (ethanol as a solvent)

The sitagliptin intermediate (R)-3-amino-1-morpholino-4-(2,4,5-trifluorophenyl)-1-butanone was synthesised through a bio-catalytic reaction carried out with the recombinant *Escherichia coli* BL21/pET28b-MS3mutl wet cells containing the recombinant expression plasmids obtained by the method in Example 3 as a bio-catalyst and the sitagliptin intermediate precursor ketone [1-morpholino-4-(2,4,5-trifluorophenyl)-1,3-butanedione] as the substrate.

Final concentration composition and catalytic conditions of a catalytic system (100 ml): 25.0 g of wet cells, 50 g/L 1-morpholino-4-(2,4,5-trifluorophenyl)-1,3-butanedione as the substrate, ethanol at a final concentration of 65% (v/v), 0.5 g/L pyridoxal phosphate, and 10 g/L isopropylamine. Reaction conditions: temperature of 45°C, stirring speed of 1,200 r/min, and reaction time of 24 hours. Under the same conditions, reaction solution without cells added was used as a blank control, and the *Escherichia coli* BL21/pET28b wet cells replaced the above recombinant *Escherichia coli* BL21/pET28b-MS3 as a negative control. Samples were taken for HPLC detection after the reaction was completed. A transformation rate of the MS3 mutant 1 for the substrate was 92.4%, and an ee value was greater than 99%.

### Example 22: Application of 1-morpholino-4-(2,4,5-trifluorophenyl)-1,3-butanedione as a substrate in synthesis of (R)-3-amino-1-morpholino-4-(2,4,5-trifluorophenyl)-1-butanone (DMF as a solvent)

The sitagliptin intermediate (R)-3-amino-1-morpholino-4-(2,4,5-trifluorophenyl)-1-butanone was synthesised through a bio-catalytic reaction carried out with the recombinant *Escherichia coli* BL21/pET28b-MS3mutl wet cells containing the recombinant expression plasmids obtained by the method in Example 3 as a bio-catalyst and the sitagliptin intermediate precursor ketone [1-morpholino-4-(2,4,5-trifluorophenyl)-1,3-butanedione] as the substrate.

Final concentration composition and catalytic conditions of a catalytic system (100 ml): 25.0 g of wet cells, 50 g/L 1-morpholino-4-(2,4,5-trifluorophenyl)-1,3-butanedione as the substrate, DMF at a final concentration of 50% (v/v), 0.5 g/L pyridoxal phosphate, and 10 g/L isopropylamine. Reaction conditions: temperature of 45°C, stirring speed of 1,200 r/min, and reaction time of 24 hours. Under the same conditions, reaction solution without cells added was used as a blank control, and the *Escherichia coli* BL21/pET28b wet cells replaced the above recombinant *Escherichia coli* BL21/pET28b-MS3 as a negative control. Samples were taken for HPLC detection after the reaction was completed. A transformation rate of the MS3 mutant 1 for the substrate was 93.1%, and an ee value was greater than 99%.

### Example 23: Application of (2Z)-4-oxo-4-[3-(trifluoromethyl)-5,6-dihydro-[1,2,4] triazolo[4,3-a]pyrazine-7(8H)-yl]-1-(2,4,5-trifluorophenyl)butan-2-one as a substrate in synthesis of sitagliptin

Sitagliptin was synthesised through a bio-catalytic reaction carried out with the recombinant *Escherichia coli* BL21/pET28b-MS3mut3 wet cells containing the recombinant expression plasmids obtained by the method in Example 3 as a bio-catalyst and the sitagliptin intermediate precursor ketone [(2Z)-4-oxo-4-[3-(trifluoromethyl)-5,6-dihydro-[1,2,4]triazolo [4,3-a]pyrazine-7(8H)-yl]-1-(2,4,5-trifluorophenyl)butan-2-one] as the substrate.

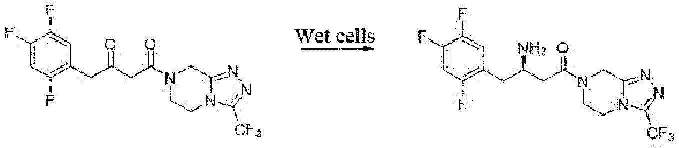

Final concentration composition and catalytic conditions of a catalytic system (100 ml): 25.0 g of wet cells, triethanolamine buffer with pH of 8-8.5, 50 g/L 2Z)-4-oxo-4-[3-(trifluoromethyl)-5,6-dihydro-[1,2,4]triazolo[4,3-a]pyrazine-7(8H)-yl]-1-(2,4 ,5-trifluorophenyl)butan-2-one as the substrate, DMSO at a final concentration of 50% (v/v), 0.5 g/L pyridoxal phosphate, and 10 g/L isopropylamine. Reaction conditions: temperature of 45°C, stirring speed of 1,200 r/min, and reaction time of 24 hours. Under the same conditions, reaction solution without cells added was used as a blank control, and the *Escherichia coli* BL21/ pET28b wet cells replaced the above recombinant *Escherichia coli* BL21/pET28b-MS3 as a negative control. Samples were taken for HPLC detection after the reaction was completed. A transformation rate of the MS3 mutant 3 for the substrate was 93.4%, and an ee value was greater than 99.5%.

### Example 24: Application of (2Z)-4-oxo-4-[3-(trifluoromethyl)-5,6-dihydro-[1,2,4] triazolo[4,3-a]pyrazine-7(8H)-yl]-1-(2,4,5-trifluorophenyl)butan-2-one as a substrate in synthesis of sitagliptin

Sitagliptin was synthesised through a bio-catalytic reaction carried out with the recombinant *Escherichia coli* BL21/pET28b-MS3mut4 wet cells containing the recombinant expression plasmids obtained by the method in Example 3 as a bio-catalyst and the sitagliptin intermediate precursor ketone [(2Z)-4-oxo-4-[3-(trifluoromethyl)-5,6-dihydro-[1,2,4]triazolo [4,3-a]pyrazine-7(8H)-yl]-1-(2,4,5-trifluorophenyl)butan-2-one] as the substrate.

Final concentration composition and catalytic conditions of a catalytic system (100 ml): 25.0 g of wet cells, 50 g/L (2Z)-4-oxo-4-[3-(trifluoromethyl)-5,6-dihydro-[1,2,4]triazolo[4,3-a]pyrazine-7(8H)-yl]-1-(2,4 ,5-trifluorophenyl)butan-2-one as the substrate, isopropanol at a final concentration of 65% (v/v), 0.5 g/L pyridoxal phosphate, and 10 g/L isopropylamine. Reaction conditions: temperature of 45°C, stirring speed of 1,200 r/min, and reaction time of 24 hours. Under the same conditions, reaction solution without cells added was used as a blank control, and the *Escherichia coli* BL21/pET28b wet cells replaced the above recombinant *Escherichia coli* BL21/pET28b-MS3 as a negative control. Samples were taken for HPLC detection after the reaction was completed. A transformation rate of the MS3 mutant 4 for the substrate was 94.5%, and an ee value was greater than 99.5%.

### Example 25: Application of (2Z)-4-oxo-4-[3-(trifluoromethyl)-5,6-dihydro-[1,2,4] triazolo[4,3-a]pyrazine-7(8H)-yl]-1-(2,4,5-trifluorophenyl)butan-2-one as a substrate in synthesis of sitagliptin (ethanol as a solvent)

Sitagliptin was synthesised through a bio-catalytic reaction carried out with the recombinant *Escherichia coli* BL21/pET28b-MS3mutl wet cells containing the recombinant expression plasmids obtained by the method in Example 3 as a bio-catalyst and the sitagliptin intermediate precursor ketone [(2Z)-4-oxo-4-[3-(trifluoromethyl)-5,6-dihydro-[1,2,4]triazolo [4,3-a]pyrazine-7(8H)-yl]-1-(2,4,5-trifluorophenyl)butan-2-one] as the substrate.

Final concentration composition and catalytic conditions of a catalytic system (100 ml): 25.0 g of wet cells, 50 g/L (2Z)-4-oxo-4-[3-(trifluoromethyl)-5,6-dihydro-[1,2,4]triazolo[4,3-a]pyrazine-7(8H)-yl]-1-(2,4 ,5-trifluorophenyl)butan-2-one as the substrate, ethanol at a final concentration of 65% (v/v), 0.5 g/L pyridoxal phosphate, and 10 g/L isopropylamine. Reaction conditions: temperature of 45°C, stirring speed of 1,200 r/min, and reaction time of 24 hours. Under the same conditions, reaction solution without cells added was used as a blank control, and the *Escherichia coli* BL21/pET28b wet cells replaced the above recombinant *Escherichia coli* BL21/pET28b-MS3 as a negative control. Samples were taken for HPLC detection after the reaction was completed. A transformation rate of the MS3 mutant 1 for the substrate was 91.8%, and an ee value was greater than 99 %.

### Example 26: Application of (2Z)-4-oxo-4-[3-(trifluoromethyl)-5,6-dihydro-[1,2,4] triazolo[4,3-a]pyrazine-7(8H)-yl]-1-(2,4,5-trifluorophenyl)butan-2-one as a substrate in synthesis of sitagliptin

Sitagliptin was synthesised through a bio-catalytic reaction carried out with the recombinant *Escherichia coli* BL21/pET28b-MS3mutl wet cells containing the recombinant expression plasmids obtained by the method in Example 3 as a bio-catalyst and the sitagliptin intermediate precursor ketone [(2Z)-4-oxo-4-[3-(trifluoromethyl)-5,6-dihydro-[1,2,4]triazolo [4,3-a]pyrazine-7(8H)-yl]-1-(2,4,5-trifluorophenyl)butan-2-one] as the substrate.

Final concentration composition and catalytic conditions of a catalytic system (100 ml): 25.0 g of wet cells, 50 g/L (2Z)-4-oxo-4-[3-(trifluoromethyl)-5,6-dihydro-[1,2,4]triazolo[4,3-a]pyrazine-7(8H)-yl]-1-(2,4 ,5-trifluorophenyl)butan-2-one as the substrate, DMF at a final concentration of 50% (v/v), 0.5 g/L pyridoxal phosphate, and 10 g/L isopropylamine. Reaction conditions: temperature of 45°C, stirring speed of 1,200 r/min, and reaction time of 24 hours. Under the same conditions, reaction solution without cells added was used as a blank control, and the *Escherichia coli* BL21/pET28b wet cells replaced the above recombinant *Escherichia coli* BL21/pET28b-MS3 as a negative control. Samples were taken for HPLC detection after the reaction was completed. A transformation rate of the MS3 mutant 1 for the substrate was 91.5%, and an ee value was greater than 99 %.

Finally, it should be noted that the above contents are merely illustrative of the technical solution of the present disclosure, but are not intended to limit the scope of protection of the present disclosure. Simple modifications or equivalent replacements made by those of ordinary skill in the art on the technical solution of the present disclosure do not depart from the essence and scope of the technical solution of the present disclosure.

## Claims

1. An omega-transaminase mutant, obtained by carrying out single-point mutation or multi-point combined mutation at positions 275, 115, and 97 of an amino acid sequence shown in SEQ ID NO.2.

2. The omega-transaminase mutant according to claim 1, **characterized in that** the mutation is one or a combination of two or more of the following: (1) mutation of glycine at the position 275 to alanine; (2) mutation of lysine at the position 115 to methionine; and (3) mutation of lysine at the position 97 to arginine.

3. A gene encoding the omega-transaminase mutant according to claim 1.

4. The encoding gene according to claim 4, **characterized in that** a nucleotide sequence of the encoding gene is as shown in SEQ ID NO.3 or SEQ ID NO.5 or SEQ ID NO.7.

5. A recombinant vector containing the gene encoding the omega-transaminase mutant according to claim 1.

6. Genetically engineered bacteria containing the gene encoding the omega-transaminase mutant according to claim 1.

7. An application of the omega-transaminase mutant according to claim 1 in preparation of sitagliptin or a sitagliptin intermediate through microbial catalysis.

8. The application according to claim 7, **characterized in that** the application is as follows: forming a reaction system with sitagliptin precursor ketone as shown in Formula (I) as a reaction substrate, a wet cell containing the omega-transaminase mutant as a bio-catalyst, a protonic polar solvent as a cosolvent, pyridoxal phosphate as a coenzyme, isopropylamine as a cosubstrate, and a triethanolamine buffer with pH of 8-9 as a reaction medium, carrying out a bio-catalytic reaction at a temperature of 30-50°C and a stirring speed of 100-800 r/min, and separating and purifying reaction liquid after the reaction, to obtain the sitagliptin intermediate as shown in Formula (II); 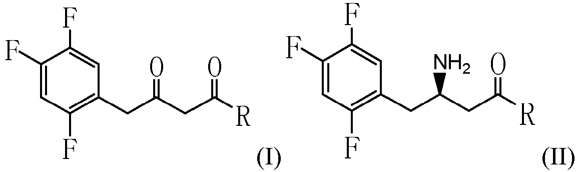 wherein, in Formula (I) and Formula (II), R is C1-C10 alkyl or alkoxy or piperidyl or morpholinyl or pyrazinyl.

9. The application according to claim 7, **characterized in that** the application is as follows: forming a reaction system with (2Z)-4-oxo-4-[3-(trifluoromethyl)-5,6-dihydro-[1,2,4]triazolo[4,3-a]pyrazine-7(8H)-yl]-1-(2,4 ,5-trifluorophenyl)butan-2-one as a reaction substrate, the wet cell containing the omega-transaminase mutant as the bio-catalyst, the protonic polar solvent as the cosolvent, the pyridoxal phosphate as the coenzyme, the isopropylamine as the cosubstrate, and the triethanolamine buffer with pH of 8-9 as the reaction medium, carrying out the bio-catalytic reaction at a temperature of 30-50°C and a stirring speed of 100-800 r/min, and separating and purifying reaction liquid after the reaction, to obtain the sitagliptin.

10. The application according to claim 8 or 9, **characterized in that** the protonic polar solvent is one or a mixture of two or more of the following: dimethyl sulfoxide, dimethyl formamide, isopropanol, and ethanol.

11. The application according to claim 8 or 9, **characterized in that** in the reaction system, a use amount of the wet cell is 10-50 g/L, a final concentration of the substrate is 50-200 g/L, a final volume concentration of the protonic polar solvent is 40-70%, the pyridoxal phosphate is 0.5-2 g/L, and the isopropylamine is 5-20 g/L.
